(19) Europäisches Patentamt
European Patent Office
Office européen des brevets

(11) **EP 3 922 174 A1**

(12) **EUROPEAN PATENT APPLICATION**

(43) Date of publication:
**15.12.2021 Bulletin 2021/50**

(21) Application number: **21178360.0**

(22) Date of filing: **08.06.2021**

(51) Int Cl.:
*A61B 5/0205* (2006.01)   *A61B 5/00* (2006.01)
*B64D 11/00* (2006.01)   *B64D 45/00* (2006.01)
*A61B 5/0507* (2021.01)   *A61B 5/08* (2006.01)
*A61B 5/113* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.06.2020   US 202063036370 P**
**07.06.2021   US 202117340323**

(71) Applicant: **B/E Aerospace, Inc.**
**Winston-Salem, NC 27105 (US)**

(72) Inventors:
• **PEARSON, Matthew R.**
**Hartford, CT 06103 (US)**
• **AGRAWAL, Piyush**
**South Windsor, CT 06074 (US)**

(74) Representative: **Dehns**
**St. Bride's House**
**10 Salisbury Square**
**London EC4Y 8JD (GB)**

(54) **AIRCRAFT PASSENGER SERVICE UNIT HEALTH MONITORING SYSTEM**

(57)   A passenger service unit health monitoring system (100) includes a cardiorespiratory sensor unit (104), a temperature sensor (102), and one or more controllers (110) having one or more processors configured to execute a set of program instructions maintained in one or more memory units, wherein the set of program instructions is configured to cause the one or more processors to: receive (302) one or more signals from at least one of the temperature sensor or the cardiorespiratory sensor unit indicative of one or more physiological states of the subject; measure (304) one or more physiological states of the subject based on the one or more signals indicative of one or more physiological states of the subject; determine (306) one or more physiological interventions based on the one or more physiological states of the subject; and transmit (308) one or more signals instructive of the one or more physiological interventions.

**FIG. 2**

## Description

CROSS-REFERENCE TO RELATED APPLICATION

The present application claims priority from U.S. Provisional Application No. 63/036,370, filed June 8, 2020.

GOVERNMENT LICENSE RIGHTS

This invention was made with government support under Task Assignment DEAR0000939 awarded by the Department of Energy. The government has certain rights in the invention.

BACKGROUND

Conventional passenger service units (PSU) installed in overhead panels generally provide passengers with a reading light, a gasper or fan outlet, and a call button (for requesting assistance from cabin crew) as elements. Such PSU's do not include features for addressing various health concerns. Airlines face significant customer confidence challenges with respect to contagious vectors; especially those spread by incidental contact or droplets. Passengers need to be confident that they are not at greater than normal risk of infection when taking a flight. Therefore, it would be advantageous to provide a device, system, and method that cures the shortcomings described above.

SUMMARY

A passenger service unit health monitoring system is described, in accordance with one or more embodiments of the present disclosure. In one embodiment, the passenger service unit health monitoring system including a temperature sensor configured to measure a bodily temperature of a human passenger. In another embodiment, the passenger service unit health monitoring system includes a cardiorespiratory sensor unit configured to measure a cardiorespiratory state of the human passenger. In another embodiment, the passenger service unit health monitoring system includes a communication circuitry. In another embodiment, the passenger service unit health monitoring system includes one or more controllers having one or more processors communicatively coupled to the temperature sensor and the cardiorespiratory sensor unit. In another embodiment, the one or more processors are configured to execute a set of program instructions maintained in one or more memory units. In another embodiment, the set of program instructions is configured to cause the one or more processors to receive one or more signals from at least one of the temperature sensor or the cardiorespiratory sensor unit. In another embodiment, the one or more signals are indicative of one or more physiological states of the human passenger.

A method for passenger health monitoring is described in accordance with one or more embodiments of the present disclosure. In one embodiment, the method includes receiving one or more signals from at least one of a temperature sensor or a cardiorespiratory sensor unit. The one or more signals may be indicative of one or more physiological states of a human passenger. In another embodiment, the signals received from the temperature sensor is indicative of a bodily temperature of the human passenger. In another embodiment, the signals received from the cardiorespiratory sensor unit is indicative of a cardiorespiratory state of the human passenger. In another embodiment, the method includes measuring one or more physiological states of the human passenger based on the one or more signals indicative of one or more physiological states of the human passenger. In another embodiment, the method includes determining one or more physiological interventions based on the one or more physiological states of the human passenger. In another embodiment, the method includes transmitting one or more signals instructive of the one or more physiological interventions.

It is to be understood that both the foregoing general description and the following detailed description are exemplary and explanatory only and should not restrict the scope of the claims. The accompanying drawings, which are incorporated in and constitute a part of the specification, illustrate exemplary embodiments of the inventive concepts disclosed herein and together with the general description, serve to explain the principles.

BRIEF DESCRIPTION OF THE DRAWINGS

The numerous advantages of the embodiments of the inventive concepts disclosed herein may be better understood by those skilled in the art by reference to the accompanying figures in which:

FIG. 1 is a simplified block diagram illustrating an aircraft passenger service unit health monitoring system, in accordance with one or more embodiments of the present disclosure.

FIG. 2 is a simplified conceptual view of a passenger service unit health monitoring system, in accordance with one or more embodiments of the present disclosure.

FIG. 3 is a process flow diagram illustrating the steps of a method of passenger health monitoring, in accordance with one or more embodiments of the present disclosure.

DETAILED DESCRIPTION

Before explaining at least one embodiment of the inventive concepts disclosed herein in detail, it is to be understood that the inventive concepts are not limited in their application to the details of construction and the arrangement of the components or steps or methodologies set forth in the following description or illustrated in the drawings. In the following detailed description of embodiments of the instant inventive concepts, numerous specific details are set forth in order to provide a more thorough understanding of the inventive concepts. However, it will be apparent to one of ordinary skill in the art having the benefit of the instant disclosure that the inventive concepts disclosed herein may be practiced without these specific details. In other instances, well-known features may not be described in detail to avoid unnecessarily complicating the instant disclosure. The inventive concepts disclosed herein are capable of other embodiments or of being practiced or carried out in various ways. Also, it is to be understood that the phraseology and terminology employed herein is for the purpose of description and should not be regarded as limiting.

As used herein a letter following a reference numeral is intended to reference an embodiment of the feature or element that may be similar, but not necessarily identical, to a previously described element or feature bearing the same reference numeral (e.g., 1, 1a, 1b). Such shorthand notations are used for purposes of convenience only, and should not be construed to limit the inventive concepts disclosed herein in any way unless expressly stated to the contrary.

Further, unless expressly stated to the contrary, "or" refers to an inclusive or and not to an exclusive or. For example, a condition A or B is satisfied by anyone of the following: A is true (or present) and B is false (or not present), A is false (or not present) and B is true (or present), and both A and B are true (or present).

In addition, use of the "a" or "an" are employed to describe elements and components of embodiments of the instant inventive concepts. This is done merely for convenience and to give a general sense of the inventive concepts, and "a" and "an" are intended to include one or at least one and the singular also includes the plural unless it is obvious that it is meant otherwise.

Finally, as used herein any reference to "one embodiment," or "some embodiments" means that a particular element, feature, structure, or characteristic described in connection with the embodiment is included in at least one embodiment of the inventive concepts disclosed herein. The appearances of the phrase "in some embodiments" in various places in the specification are not necessarily all referring to the same embodiment, and embodiments of the inventive concepts disclosed may include one or more of the features expressly described or inherently present herein, or any combination of sub-combination of two or more such features, along with any other features which may not necessarily be expressly described or inherently present in the instant disclosure.

Broadly, embodiments of the inventive concepts disclosed herein are directed to a passenger service unit health monitoring system. The aircraft passenger service unit (PSU) health monitoring system may be disposed within one or more PSUs of an aircraft. The PSU health monitoring system may provide in-flight measurement of vital signs of one or more passengers. The PSU health monitoring system may alert aircraft crew of vital signs of one or more passengers that may be indicative of one or more contagious infections so that adequate precautions (e.g., social distancing, aircraft diversion, and the like) may be implemented. The system may include one or more controllers configured to determine one or more physiological states of a passenger. The system may include one or more physiological state sensors for detecting one or more signals indicative of one or more physiological states of a passenger. Such physiological states may include one or more of a cardiorespiratory state or a temperature. One or more components of the system may be communicatively coupled to at least one of a network or a remote server for transmitting the sensed information and/or a signals instructive of an intervention.

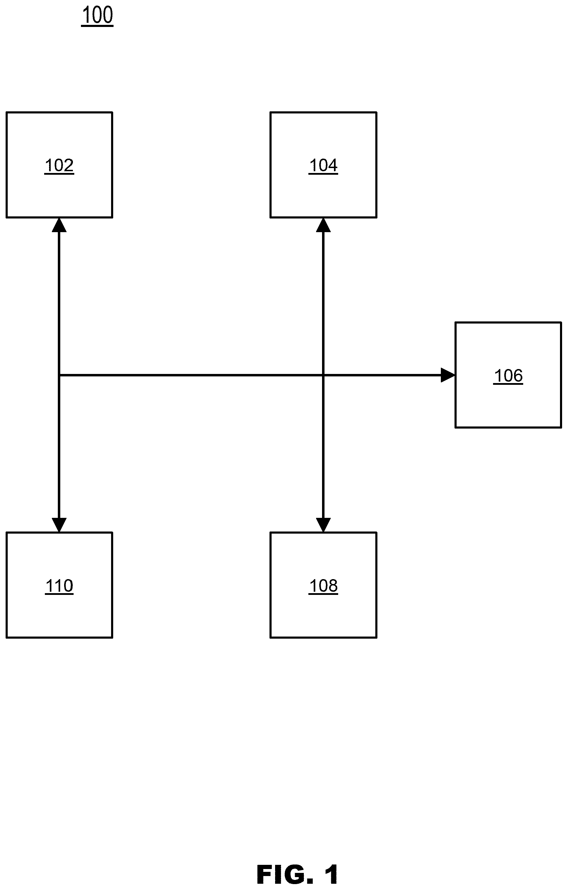

FIG. 1 illustrates a simplified block diagram illustrating a passenger service unit health monitoring system 100 in accordance with one or more embodiments of the present disclosure. The passenger service unit health monitoring system 100 may include one or more of a temperature sensor 102, a cardiorespiratory sensor unit 104, a communication circuitry 106, a memory unit 108, or one or more controllers 110. One or more components of the passenger service unit health monitoring system 100 may be communicatively coupled to one or more other components of the passenger service unit health monitoring system 100 in any manner known in the art. For example, the temperature sensor 102, the cardiorespiratory sensor unit 104, the communication circuitry 106, and the one or more controllers 110 may be communicatively coupled to each other and other components via a wireline connection (e.g., copper wire, fiber optic cable, soldered connection, and the like) or a wireless connection (e.g., RF coupling, IR coupling, data network communication (e.g., WiFi, WiMAX, Bluetooth, and the like)).

The temperature sensor 102 may include any sensor type known in the art to be suitable for measuring or determining the bodily temperature of a human subject within an ambient environment. For example, the temperature sensor 102 may include, but is not limited to, one or more infrared sensors, including an infrared sensor configured to include one or more focal plane arrays, one or more hyperspectral sensor units, and/or one or more thermal emission scanning units. As used herein, the term "ambient environment" may refer to, but is not limited to, communal spaces where it is typical for human subjects to congregate, including, without limitation, the passenger cabin of an aircraft.

The temperature sensor 102 may be configured to measure and/or determine the bodily temperature of a human subject within an ambient environment. For example, the temperature sensor 102 may be configured to generate one or more signals (e.g., radiometric infrared images) indicative of bodily temperature based on a skin temperature of one or more human subjects. The one or more controllers 110 may be configured to direct the temperature sensor 102 to generate one or more signals indicative of the bodily temperature of a particular region of interest of the human body (e.g., the face of a human subject).

The cardiorespiratory sensor unit 104 may include any sensor type known in the art to be suitable for measuring or determining one or more cardiorespiratory states (e.g., breathing rate, respiration pattern, and the like) of a human subject within an ambient environment. For example, the cardiorespiratory sensor unit 104 may include any device configured to transmit electromagnetic waves from a transmitter and to receive electromagnetic waves at a receiver. In another example, the cardiorespiratory sensor unit 104 may include a radar sensor unit, including, without limitation, a radar sensor unit configured to transmit electromagnetic radiation from a transmitter and to receive electromagnetic radiation at a receiver. By way of an additional example, the cardiorespiratory sensor unit 104 may include any low-cost radar device known to be suitable to measure or determine one or more cardiorespiratory states of a human subject. As used herein, the term "ambient environment" may refer to, but is not limited to, communal spaces where it is typical for human subjects to congregate, including, without limitation, the passenger cabin of an aircraft.

The cardiorespiratory sensor unit 104 may be configured to measure and/or determine one or more cardiorespiratory states of a human subject within an ambient environment. For example, the cardiorespiratory sensor unit 104 may be configured to transmit one or more electromagnetic waves within an ambient environment containing one or more human subjects. In some embodiments, the electromagnetic waves may include an operating frequency or a range of operating frequencies which are certified for in-flight use (e.g., in the radar frequency band). The cardiorespiratory sensor unit 104 may further be configured to receive one or more electromagnetic waves following one or more interactions of the one or more electromagnetic waves with one or more human subjects. The cardiorespiratory sensor unit 104 may generally be considered a contactless sensor for respiration patterns. The cardiorespiratory sensor unit 104 may detect a respiration pattern of a human subject. During a breath cycle, heartbeats may be detected from the respiration pattern.

The one or more controllers 110 may be configured to determine one or more cardiorespiratory states of the one or more human subjects based on one or more signals generated by the cardiorespiratory sensor unit 104. The one or more controllers 110 may include one or more processors. Such processors may be configured to execute any of the various processes or methods described herein. For example, the one or more controllers 110 may process one or more signals from the cardiorespiratory sensor unit 104 to determine one or more phase changes of the one or more electromagnetic waves transmitted by the cardiorespiratory sensor unit 104. In this regard, the one or more controllers 110 may determine minor movements of one or more portions (e.g., a chest or a heart) of the one or more human subjects, where the minor movements may be indicative of one or more cardiorespiratory states of the one or more human subjects. By way of an additional example, the one or more controllers 110 may be configured to determine one or more distance differentials between the cardiorespiratory sensor unit 104 and the one or more human subjects. In this regard, the cardiorespiratory sensor unit 104 may be configured to measure one or more signals indicative of respiration rate by comparing the difference between a first measured distance between the cardiorespiratory sensor unit 104 and one or more human subjects and a second measured distance between the cardiorespiratory sensor unit 104 and the one or more human subjects. Put another way, the cardiorespiratory sensor unit 104 may measure the distance between the passenger service unit health monitoring system 100 and one or more human subjects at a first point in time at which at least one of the one or more human subject inhales and at a second point in time at which the at least one human subject exhales. A phase of the radar returns may be sensitive to motions, based on the wavelength of the radar signal. Accordingly, the one or more controllers 110 may be configured to determine one or more physiological states of the one or more human subjects using the following formula, where Dexhale represents the distance between the passenger service unit health monitoring system 100 and a human subject at the point in time at which the human subject exhales, and where Dinhale represents the distance between the passenger service unit health monitoring system 100 and the human subject at the point in time at which the human subject exhales:

$$\Delta D = D_{exhale} - D_{inhale}$$

Chest motion during inhaling and exhaling may introduce periodicity in the phase of the radar returns. A spectral analysis and periodicity test on the phase may be performed to detect the respiration.

The one or more physiological states may be indicative of various abnormal conditions. For example, the physiological states may indicate one or more of an infection, a heart attack, or a stroke. In this regard, various physiological states may be indicative of abnormal conditions. For instance, a human with an infection, heart attack, or stroke may exhibit a breathing pattern disorder or a change in temperature. By determining the physiological state of the user as being abnormal, emergency medical attention may be provided to the passenger.

The one or more controllers 110 may also be configured to generate one or more plots for display to a user via one or more user interfaces based on the one or more signals generated by at least one of the temperature sensor 102 or the cardiorespiratory unit 104.

The one or more processors of the one or more controllers 110 may include any one or more processing elements known in the art. In general, the term "processor" may be broadly defined to encompass any device having one or more processing elements, which execute program instructions from a non-transitory memory medium (i.e., memory). In one embodiment, the one or more processors may include any microprocessor-type computational device configured to execute software algorithms and/or instructions. In general, the processor may be broadly defined to encompass any device having data processing or logic capabilities. It should be recognized that the steps described throughout the present disclosure may be carried out by a single passenger service unit health monitoring system 100 or by a plurality of passenger service unit health monitoring system 100.

The communication circuitry 106 may include any communication circuitry known in the art. The communication circuitry 106 may include one or more components that may be configured to transmit data in a manner that combines elements of any configuration of transmission known in the art. For instance, the communication circuitry 106 may include wireline-based communication circuitry (e.g., DSL-based interconnection, cable-based interconnection, T9-based interconnection, fiber-optic lines, and the like). In another instance, the communication circuitry 106 may include wireless-based communication circuitry, such as one or more of GSM, GPRS, CDMA, EV-DO, EDGE, WiMAX, 3G, 4G, LTE, 5G, 6G, Wi-Fi protocols, LoRa, customized RF protocol, and the like. The communication circuitry 106 may be configured to transmit one or more signals to a user interface.

The memory unit 108 may include a memory unit or storage medium known in the art to be suitable for storing program instructions executable by the one or more processors of the one or more controllers 110. For example, the memory unit 108 may include a non-transitory memory medium. For instance, the memory unit 108 may include, but is not limited to, a read-only memory (ROM), a random-access memory (RAM), a magnetic or optical memory device, a magnetic tape, a solid-state drive, and the like. In another embodiment the memory unit 108 may be configured for storing one or more signals received from the temperature sensor 102 and/or the cardiorespiratory sensor unit 104. It is further noted that the memory unit 108 may be housed in a common housing with the one or more processors. In an alternative embodiment, the memory unit 108 may be located in a remote server. The memory unit 108 may maintain program instructions for causing the one or more processors to carry out various steps described in the present disclosure.

The passenger service unit health monitoring system 100 may further include a mounting mechanism (not depicted). By the mounting mechanism, the passenger service unit health monitoring system 100 may mount above an aircraft seat. For example, the mounting mechanism may include, but is not limited to, a fastener, a screw, a snap, a clamp, a clasp, a clip, a pin, a hook-and-loop fastener, a retaining ring, a rivet, an interference fit, or any other fastening means.

In embodiments, the passenger service unit health monitoring system 100 is configured to fit within one or more existing recesses of an aircraft. For example, a passenger seating area may include one or more recesses by which a spotlight may be installed. Such recess may include, but is not limited to, dome lighting. In some instances, the aircraft seating area may include three domes, by which one or more may be replaced with the passenger service unit health monitoring system 100. The passenger service unit health monitoring system 100 may be configured to mount within the recess for the spotlight. In this regard, the passenger service unit health monitoring system 100 may be packaged with a form factor equivalent to an overhead reading light while also performing the functionality of measuring breathing patterns, heart rates, temperature, or other suitable bioinformatic information.

Although the controller 110 is described as determining the physiological states of the human passenger based on the one or more signals indicative of one or more physiological states of the human passenger, this is not intended as a limitation on the present disclosure. In this regard, the controller 110 may receive the one or more signals indicative of one or more physiological states of the human passenger from one or more of the temperature sensor or the cardiorespiratory sensor unit. The controller 110 may then transmit the signals (e.g., by the communication circuitry 106) to one or more remote controllers. The one or more remote controllers may then determine the physiological states of the human passenger based on the one or more signals indicative of one or more physiological states of the human passenger. The remote controller may further determine one or more physiological interventions based on the one or more physiological states of the human passenger.

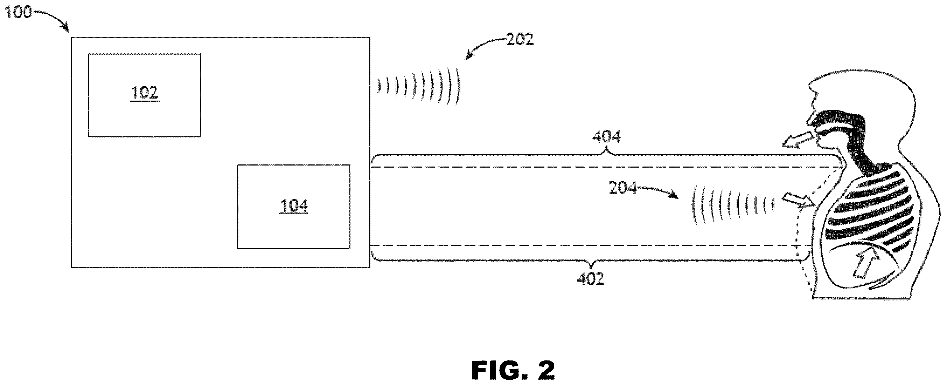

FIG. 2 is an exemplary embodiment of a simplified conceptual view of a passenger service unit health monitoring system 100, in accordance with one or more embodiments of the present disclosure. The one or more controllers 110 may be configured to determine one or more cardiorespiratory states of one or more human subjects 206 (also known as a human passenger) based on one or more signals generated by the passenger service unit health monitoring system 100. For example, the one or more controllers 110 may cause the cardiorespiratory sensor unit 104 and/or the temperature sensor 102 to transmit one or more transmitting signals 202 toward the one or more human subjects 206. As an additional example, the passenger service unit health monitoring system 100 may collect one or more reflected signals 204 following interaction of the one or more transmitting signals 202 with the one or more human subjects 206. For example, the passenger service unit health monitoring system 100 may transmit one or more transmitting signals 202 along a first distance 404 toward the one or more human subjects 206. As an additional example, the passenger service unit health monitoring system 100 may receive the one or more reflected signals 204 that have interacted with the one or more human subjects 206 and have been reflected along a second distance 402 toward the passenger service unit health monitoring system 100. In this regard, the passenger service unit health monitoring system 100 (e.g., via the one or more controllers 110) may determine minor movements of one or more portions (e.g., a chest or a heart) of the one or more human subjects 206, where the minor movements may be indicative of one or more cardiorespiratory states of the one or more human subjects 206. By way of an additional example, the one or more controllers 110 may be configured to determine one or more distance differentials between the cardiorespiratory sensor unit 104 and the one or more human subjects 206. In this regard, the cardiorespiratory sensor unit 104 may be configured to measure one or more signals indicative of respiration rate by comparing the difference between the first distance 404 and the second distance 402.

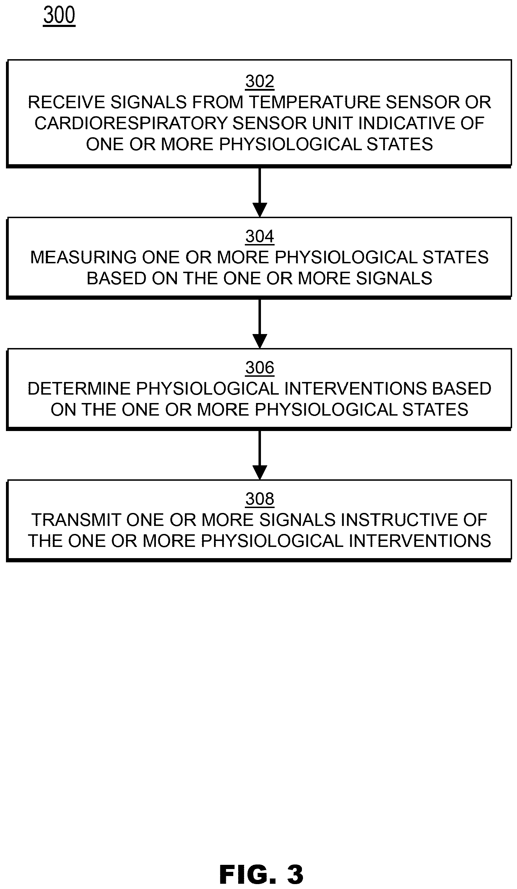

FIG. 3 is a process flow diagram illustrating the steps of a method 300 of passenger health monitoring, in accordance with one or more embodiments of the present disclosure. The embodiments and the enabling technologies described previously herein in the context of the system 100 should be interpreted to extend to the method 300. It is further recognized, however, that the method 300 is not limited to the system 100.

In Step 302, one or more signals indicative of one or more physiological states are measured. For example, at least one of the temperature sensor 102 or the cardiorespiratory sensor unit 104 may transmit one or more transmitting signals 202 toward the one or more human subjects 206. By way of an additional example, the passenger service unit health monitoring system 100 may receive one or more reflected signals 204 reflected from one or more of the human subjects 206.

In Step 304, one or more physiological states are determined based on the one or more reflected signals 204. For example, the one or more controllers 110 may determine the difference between the first distance 404 and the second distance 402. In this regard, the passenger service unit health monitoring system 100 may determine one or more physiological states, including, without limitation, a respiration pattern of the one or more human subjects 206. As an additional example, the one or more controllers 110 may determine one or more phase changes of at least one of the transmitting signals 202 or the reflected signals 204. In this regard, the one or more controllers 110 may determine a cardiorespiratory pattern of the one or more human subjects 206. As an additional example, the one or more controllers 110 may determine one or more bodily temperatures of the one or more human subjects 206 based on one or more transmitting signals 202 transmitted by the temperature sensor 102. In this regard, the data received from the various sensors may be parsed.

In Step 306, one or more physiological interventions are determined based on the one or more physiological states of the one or more human subjects 206. For example, the one or more controllers 110 may determine one or more precautionary actions (e.g., self-isolation, social distancing, aircraft diversion) based on the one or more physiological states of the one or more human subjects 206. The one or more physiological interventions may include any appropriate act or series of acts intended to reduce the spread of a contagious vector among passengers and crewmembers of an aircraft. In this regard, the passenger may be flagged as a passenger-of-interest. Similarly, the physiological intervention may include providing emergency medical attention (e.g., providing a defibrillator, etc.).

In Step 308, one or more physiological interventions are transmitted. For example, the one or more controllers 110 (e.g., via the communication circuitry 106 or a user interface) may transmit the one or more determined physiological interventions to a user or remote location. In this regard, the passenger service unit health monitoring system 100 may be configured to alert a user (e.g., an airline crew member) of the determined physiological intervention so that appropriate steps may be taken, including, for example, aircraft diversion for medical attention. By way of an additional example, the one or more controllers 110 may be configured to transmit one or more signals indicative of the one or more physiological interventions to a network or a remote server.

All of the methods described herein may include storing results of one or more steps of the method embodiments in memory. The results may include any of the results described herein and may be stored in any manner known in the art. The memory may include any memory described herein or any other suitable storage medium known in the art. After the results have been stored, the results can be accessed in the memory and used by any of the method or system embodiments described herein, formatted for display to a user, used by another software module, method, or system, and the like. Furthermore, the results may be stored "permanently," "semi-permanently," temporarily," or for some period of time. For example, the memory may be random access memory (RAM), and the results may not necessarily persist indefinitely in the memory. It is further contemplated that each of the embodiments of the method described above may include any other step(s) of any other method(s) described herein. In addition, each of the embodiments of the method described above may be performed by any of the systems described herein.

Referring generally again to FIGS. 1-3.

One skilled in the art will recognize that the herein described components operations, devices, objects, and the discussion accompanying them are used as examples for the sake of conceptual clarity and that various configuration modifications are contemplated. Consequently, as used herein, the specific exemplars set forth and the accompanying discussion are intended to be representative of their more general classes. In general, use of any specific exemplar is intended to be representative of its class, and the non-inclusion of specific components, operations, devices, and objects should not be taken as limiting.

With respect to the use of substantially any plural and/or singular terms herein, those having skill in the art can translate from the plural to the singular and/or from the singular to the plural as is appropriate to the context and/or application. The various singular/plural permutations are not expressly set forth herein for sake of clarity.

It is believed that the inventive concepts disclosed herein and many of their attendant advantages will be understood by the foregoing description of embodiments of the inventive concepts disclosed, and it will be apparent that various changes may be made in the form, construction, and arrangement of the components thereof without departing from the broad scope of the inventive concepts disclosed herein or without sacrificing all of their material advantages; and individual features from various embodiments may be combined to arrive at other embodiments. The form herein before described being merely an explanatory embodiment thereof, it is the intention of the following claims to encompass and include such changes. Furthermore, any of the features disclosed in relation to any of the individual embodiments may be incorporated into any other embodiment.

**Claims**

1. A passenger service unit health monitoring system (100), comprising:

   a temperature sensor (102) configured to measure a bodily temperature of a human passenger (206);
   a cardiorespiratory sensor unit (104) configured to measure a cardiorespiratory state of the human passenger;
   a communication circuitry (106); and
   one or more controllers (110) having one or more processors communicatively coupled to the temperature sensor (102) and the cardiorespiratory sensor unit (104), wherein the one or more processors are configured to execute a set of program instructions maintained in one or more memory units, wherein the set of program instructions is configured to cause the one or more processors to:

      receive (302) one or more signals from at least one of the temperature sensor or the cardiorespiratory sensor unit, the one or more signals indicative of one or more physiological states of the human passenger.

2. The passenger service unit health monitoring system of Claim 1, wherein the one or more processors are further configured to:

   determine one or more physiological states of the human passenger based on the one or more signals indicative of the one or more physiological states of the human passenger;
   determine (306) one or more physiological interventions based on the one or more physiological states of the human passenger; and
   transmit (308) one or more signals instructive of the one or more physiological interventions via the communication circuitry.

3. The passenger service unit health monitoring system of Claim 2, wherein the communication circuitry (106) is communicatively coupled to at least one of additional systems for passenger health monitoring or a remote server, wherein the controller is configured to transmit one or more signals instructive of the one or more physiological interventions to the at least additional systems for passenger health monitoring or the remote server.

4. The passenger service unit health monitoring system of Claim 1 or 2, wherein the communication circuitry is communicatively coupled to at least one of an additional system for passenger health monitoring or a remote server, wherein the controller is configured to transmit the one or more signals from at least one of the temperature sensor or the cardiorespiratory sensor unit to the at least one of the additional systems for passenger health monitoring or the remote server.

5. The passenger service unit health monitoring system of any preceding Claim, wherein the temperature sensor comprises an infrared sensor.

6. The passenger service unit health monitoring system of any preceding Claim, wherein the cardiorespiratory sensor unit comprises a radar unit.

7. The passenger service unit health monitoring system of any preceding Claim, wherein the one or more physiological states of the human passenger comprise at least one of the cardiorespiratory state or the bodily temperature.

8. The passenger service unit health monitoring system of any preceding Claim, wherein the physiological state of the passenger is indicative of at least one of an infection, a heart attack, or a stroke.

9. The passenger service unit health monitoring system of any preceding Claim, wherein the cardiorespiratory state includes at least one of a heart rate of the human passenger, a respiration rate of the human passenger, or a respiration pattern of the human passenger.

10. The passenger service unit health monitoring system of any preceding Claim, wherein the passenger service unit health monitoring system is configured to fit within a recess defined for an overhead reading light.

11. A method (300) for passenger health monitoring, comprising:

> receiving (302) one or more signals from at least one of a temperature sensor (102) or a cardiorespiratory sensor unit (104) indicative of one or more physiological states of a human passenger (206);
> measuring (304) one or more physiological states of the human passenger based on the one or more signals indicative of the one or more physiological states of the human passenger;
> determining (306) one or more physiological interventions based on the one or more physiological states of the human passenger; and
> transmitting (308) one or more signals instructive of the one or more physiological interventions.

12. The method of Claim 11, wherein the signals received from the temperature sensor (102) is indicative of a bodily temperature of the human passenger, wherein the temperature sensor comprises an infrared sensor.

13. The method of claim 11, wherein the signals received from the cardiorespiratory sensor unit (104) is indicative of a cardiorespiratory state of the human passenger, wherein the cardiorespiratory sensor unit comprises a radar unit.

14. The method of claim 13, wherein the cardiorespiratory state includes at least one of a heart rate of the human passenger, a respiration rate of the human passenger, or a respiration pattern of the human passenger.

15. The method of claim 11, 12, 13, or 14, wherein the physiological state of the passenger is indicative of at least one of an infection, a heart attack, or a stroke.

100

| 102 |

| 104 |

| 106 |

| 110 |

| 108 |

**FIG. 1**

**FIG. 2**

<u>300</u>

```
┌─────────────────────────────────────────┐
│                  302                     │
│  RECEIVE SIGNALS FROM TEMPERATURE        │
│  SENSOR OR CARDIORESPIRATORY SENSOR      │
│  UNIT INDICATIVE OF ONE OR MORE          │
│  PHYSIOLOGICAL STATES                    │
└─────────────────────────────────────────┘
                     │
                     ▼
┌─────────────────────────────────────────┐
│                  304                     │
│  MEASURING ONE OR MORE PHYSIOLOGICAL     │
│  STATES BASED ON THE ONE OR MORE SIGNALS │
└─────────────────────────────────────────┘
                     │
                     ▼
┌─────────────────────────────────────────┐
│                  306                     │
│  DETERMINE PHYSIOLOGICAL INTERVENTIONS   │
│  BASED ON THE ONE OR MORE PHYSIOLOGICAL  │
│  STATES                                  │
└─────────────────────────────────────────┘
                     │
                     ▼
┌─────────────────────────────────────────┐
│                  308                     │
│  TRANSMIT ONE OR MORE SIGNALS            │
│  INSTRUCTIVE OF THE ONE OR MORE          │
│  PHYSIOLOGICAL INTERVENTIONS             │
└─────────────────────────────────────────┘
```

**FIG. 3**

Europäisches
Patentamt

European
Patent Office

Office européen
des brevets

# EUROPEAN SEARCH REPORT

Application Number

EP 21 17 8360

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/108649 A1 (KNELLER HEIDI J [US] ET AL) 30 April 2009 (2009-04-30) | 1,2,5,7, 10-12 | INV. A61B5/0205 |
| Y | * paragraph [0028] - paragraph [0035] * <br> * paragraph [0053] - paragraph [0062] * <br> * figures 1-3,10,14 * | 3,4,6,8, 9,13,15 | A61B5/00 <br> B64D11/00 <br> B64D45/00 |
| X | US 2013/338857 A1 (SAMPIGETHAYA RADHAKRISHNA G [US]) 19 December 2013 (2013-12-19) | 11,14 | ADD. A61B5/0507 A61B5/08 |
| Y | * paragraph [0024] - paragraph [0056] * | 3,4,9 | A61B5/113 |
| A | * paragraph [0080] - paragraph [0082] * <br> * paragraph [0086] - paragraph [0094] * <br> * figures 1,4,6 * | 2 | |
| Y | US 2010/036269 A1 (FERREN BRAN [US] ET AL) 11 February 2010 (2010-02-11) <br> * paragraph [0108] - paragraph [0110] * <br> * paragraphs [0134], [0343] * <br> * figures 8,24 * | 6,8,13, 15 | |
| A | US 2020/031474 A1 (SIVIGNON SÉBASTIEN [FR] ET AL) 30 January 2020 (2020-01-30) <br> * paragraph [0054] - paragraph [0087] * <br> * paragraph [0143] - paragraph [0165] * <br> * figure 1 * | 6,13 | TECHNICAL FIELDS SEARCHED (IPC) <br><br> A61B <br> B64D |

The present search report has been drawn up for all claims

| Place of search | Date of completion of the search | Examiner |
|---|---|---|
| The Hague | 25 October 2021 | Görlach, Tobias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 922 174 A1**

## ANNEX TO THE EUROPEAN SEARCH REPORT
## ON EUROPEAN PATENT APPLICATION NO.

EP 21 17 8360

This annex lists the patent family members relating to the patent documents cited in the above-mentioned European search report.
The members are as contained in the European Patent Office EDP file on
The European Patent Office is in no way liable for these particulars which are merely given for the purpose of information.

25-10-2021

| Patent document cited in search report | | | Publication date | Patent family member(s) | | | Publication date |
|---|---|---|---|---|---|---|---|
| US 2009108649 | A1 | | 30-04-2009 | US | 2009108649 | A1 | 30-04-2009 |
| | | | | US | 2011068227 | A1 | 24-03-2011 |
| US 2013338857 | A1 | | 19-12-2013 | US | 2013338857 | A1 | 19-12-2013 |
| | | | | WO | 2013187985 | A1 | 19-12-2013 |
| US 2010036269 | A1 | | 11-02-2010 | NONE | | | |
| US 2020031474 | A1 | | 30-01-2020 | CN | 110891860 | A | 17-03-2020 |
| | | | | EP | 3606819 | A1 | 12-02-2020 |
| | | | | FR | 3065104 | A1 | 12-10-2018 |
| | | | | JP | 6934065 | B2 | 08-09-2021 |
| | | | | JP | 2020516522 | A | 11-06-2020 |
| | | | | US | 2020031474 | A1 | 30-01-2020 |
| | | | | WO | 2018185332 | A1 | 11-10-2018 |

For more details about this annex : see Official Journal of the European Patent Office, No. 12/82

**REFERENCES CITED IN THE DESCRIPTION**

**Patent documents cited in the description**

- US 63036370